Europäisches Patentamt

European Patent Office

Office européen des brevets

(19)

(11) **EP 1 559 444 A1**

(12) **EUROPEAN PATENT APPLICATION**
published in accordance with Art. 158(3) EPC

(43) Date of publication:
**03.08.2005 Bulletin 2005/31**

(51) Int Cl.7: **A61M 21/02**

(21) Application number: **03780745.0**

(86) International application number:
**PCT/JP2003/016001**

(22) Date of filing: **12.12.2003**

(87) International publication number:
**WO 2004/054649 (01.07.2004 Gazette 2004/27)**

(84) Designated Contracting States:
**AT BE BG CH CY CZ DE DK EE ES FI FR GB GR
HU IE IT LI LU MC NL PT RO SE SI SK TR**
Designated Extension States:
**AL LT LV MK**

(30) Priority: **12.12.2002 JP 2002383239
04.08.2003 JP 2003205828**

(71) Applicants:
• **Ito, Hidenori**
 **Nagoya-shi, Aichi 461-0018 (JP)**
• **Kato, Shohei**
 **Nagoya-shi, Aichi 468-0011 (JP)**

• **Mizuno, Masaki**
 **Nagoya-shi, Aichi 466-0043 (JP)**

(72) Inventors:
• **Ito, Hidenori**
 **Nagoya-shi, Aichi 461-0018 (JP)**
• **Kato, Shohei**
 **Nagoya-shi, Aichi 468-0011 (JP)**
• **Mizuno, Masaki**
 **Nagoya-shi, Aichi 466-0043 (JP)**

(74) Representative: **TBK-Patent
Bavariaring 4-6
D-80336 München (DE)**

(54) **SOUND GENERATION METHOD, COMPUTER-READABLE STORAGE MEDIUM, STAND-ALONE TYPE SOUND GENERATION/REPRODUCTION DEVICE, AND NETWORK DISTRIBUTION TYPE SOUND GENERATION/REPRODUCTION SYSTEM**

(57)     An object is to provide a sound generation method for basic information having chaos and fractal, so that a sound is generated by converting basic information having chaos and fractal into data that are numerically operable, by calculating characteristics of chaos and fractal from the basic information, which has chaos and fractal, and by applying a generation rule to the data of chaos and fractal.

This objective is achieved by a sound generation method for basic information having chaos and fractal, the method comprising a basic information converting process, which converts basic information having chaos and fractal into data that are numerically operable, a chaotic space generating process, which calculates a chaos attractor and a fractal feature on the basis of the data converted by the basic information converting process and generates a chaotic space, a fractal space generating process, which generates a fractal space, and a sound generating process, which generates a sound file, in compliance with a predetermined sound generation rule, from the data in the chaotic space and the fractal space generated by the chaotic space generating process and the fractal space generating process.

Fig. 1

**Description**

TECHNICAL FIELD OF THE INVENTION

The present invention relates to a sound generation method that uses basic information having characteristics of chaos and/or fractal and that thereby generates a sound having those characteristics. Sounds generated in this way are beneficial to us, human body for maintaining good health condition.

Also, the present invention relates to a computer-readable storage medium on which a sound generation program based on the above sound generation method is stored, and it also relates to a stand-alone sound generation and playback apparatus and a network-communicative sound generation and playback system, each of which generates a sound that agrees to the above-mentioned purpose.

BACKGROUND OF THE INVENTION

As far as conventional methods of sound generation are concerned, compact discs carrying music with a theme of "healing", i.e., so-called healing music, exist in large numbers. For example, there is α-wave music intended by composers and arrangers to induce α brain waves on the listeners, and there are healing sounds, which are recorded natural sounds.

On the other hand, non-linear processing methods in which signals from the living human body are processed by a non-linear processing on the basis of the chaos theory have come to our attention recently. There is a disclosure of a technique for diagnosing the health condition in which a determination is made whether or not the pulse wave and the heartbeat as signals satisfy conditions of chaos (for example, refer to Japanese Laid-Open Patent Publication No. H4(1992)-208136 (pages 2 to 8)). This is a utilization of the knowledge that the pulse wave and the heartbeat as signals coming from a healthy living body have characteristics of chaos. In general, for certain data to be chaotic, conditions such as the fractal dimension being a non-integer and the maximum Lyapunov number being plus are to be satisfied. In addition, as a technique to diagnose objectively the mental and physical condition of a person, a technique that uses a chaos attractor and a Lyapunov index is disclosed (for example, refer to Japanese Laid-Open Patent Publication No. H6(1994)-54813 (pages 2 - 6).

As a method for evaluating the mental and physical condition of a human being quantitatively, there is a brain wave analysis. It is said generally that when a person relaxes, an α-wave appears, and that when a person gets excited, a β wave appears. As for evaluating the human mental condition, there is a disclosure of a technique that evaluates the mental condition by determining average power values of these two kinds of brain-wave bands and by calculating the ratio of the values (for example, refer to Japanese Laid-Open Patent Publication No. 2002-577 (pages 3 to 5 and FIG. 5).

However, because sounds generated in conventional sound generation methods are controlled by composers and arrangers, it is not possible to generate a sound on the basis of basic information that has a chaotic characteristic such as physiological information coming from an individual living body.

It is an object of the present invention to provide a sound generation method that uses basic information having characteristics of chaos and/or fractal. In this method, basic information is converted into computable data, and then, the characteristics of chaos and/or fractal are quantified, and a sound generation rule is applied on the quantified characteristics to generate a sound.

Furthermore, it is another object of the present invention to provide a sound generation method that modifies a generated sound to a more comforting sound by feeding back the result of an evaluation of the generated sound.

Also, it is yet another object of the present invention to provide a computer-readable storage medium on which a sound generation program based on the above sound generation method is stored, and also to provide a stand-alone sound generation and playback apparatus and a network-communicative sound generation and playback system, each of which measures a physiological signal of a person who provides information and plays a generated sound.

DISCLOSURE OF THE INVENTION

The present invention is described as follows.

1. A sound generation method comprising: a basic information converting process, where basic information having a chaos is converted to data that are numerically operable; a chaotic space generating process, where a chaotic space is generated by calculating a chaos attractor on the basis of the data, which have been converted by the basic information converting process; and a sound generating process, where a sound file is generated from the data in the chaotic space, which has been generated by the chaotic space generating process, in compliance with a predetermined sound generation rule.

2. A sound generation method comprising: a basic information converting process, where basic information having a fractal is converted to data that are numerically operable; a fractal space generating process, where a fractal space is generated by extracting a fractal feature on the basis of the data, which have been converted by the basic information converting process; and a sound generating process, where a sound file is generated from the fractal space, which has been generated by the fractal space generating process, in compliance with a predetermined sound generation rule.

3. A sound generation method comprising: a physiological signal converting process, where a signal chronologically generated from an individual informant is converted to data that are numerically operable; a chaotic space generating process, where a chaotic space is generated by calculating a chaos attractor on the basis of the data, which have been converted by the physiological signal converting process; and a sound generating process, where a sound file adapted for the informant is generated from the data in the chaotic space, which has been generated by the chaotic space generating process, in compliance with a predetermined sound generation rule.

4. A sound generation method comprising: a physiological signal converting process, where a signal chronologically generated from an individual informant is converted to data that are numerically operable; a fractal space generating process, where a fractal space is generated by extracting a feature of self-similarity on the basis of the data, which have been converted by the physiological signal converting process; and a sound generating process, where a sound file adapted for the informant is generated from the fractal space, which has been generated by the fractal space generating process, in compliance with a predetermined sound generation rule.

5. The sound generation method described in above 3, wherein the physiological signal converting process comprises: a physiological signal measuring process, which measures a physiological signal; a frequency-analyzing process, which calculates the physiological signal data measured by the physiological signal measuring process as numerical data for a plurality of frequency bands; and a sound generating process, which corresponds to a nerve-descriptive characteristic of the living body of the individual informant on the basis of the frequency-analyzing process.

6. The sound generation method described in above 4, wherein the physiological signal converting process comprises: a physiological signal measuring process, which measures a physiological signal; a frequency-analyzing process, which calculates the physiological signal data measured by the physiological signal measuring process as numerical data for a plurality of frequency bands; and a sound generating process, which corresponds to a nerve-descriptive characteristic of the living body of the individual informant on the basis of the frequency-analyzing process.

7. The sound generation method described in above 3, wherein the chaotic space generating process comprises: a condition-evaluating process, which evaluates the condition of the mind and body of the informant by comparing the numerical data, which have been calculated by the frequency-analyzing process from the nerve-descriptive characteristic of the living body of the individual informant; and a section-changing process, which changes a plane that cuts through the chaos attractor, in correspondence with the evaluation by the condition-evaluating process.

8. The sound generation method described in above 4, wherein the fractal space generating process comprises: a condition-evaluating process, which evaluates the condition of the mind and body of the informant by comparing the numerical data, which have been calculated by the frequency-analyzing process from the nerve-descriptive characteristic of the living body of the individual informant; and a scaling width modifying process, which modifies the scaling width for extracting a fractal feature, in correspondence with the evaluation by the condition-evaluating process.

9. The sound generation method described in above 3, wherein the sound generating process comprises: a condition-inputting process, which has an interface to enable communication with the informant providing the physiological signal so that conditions for the sound generation can be input; and a generation-rule setting process, which sets the sound generation rule in compliance with the conditions input by the condition-inputting process, so that the sound generating process generates the sound file in compliance with the generation rule, which has been set by the generation-rule setting process.

10. The sound generation method described in above 4, wherein the sound generating process comprises: a condition-inputting process, which has an interface to enable communication with the informant providing the physiological signal so that conditions for the sound generation can be input; and a generation-rule setting process, which sets the sound generation rule in compliance with the conditions input by the condition-inputting process, so that the sound generating process generates the sound file in compliance with the generation rule, which has been set by the generation-rule setting process.

11. The sound generation method described in above 7, wherein the condition-evaluating process evaluates the condition of the mind and body by the ratio of $\alpha$-wave appearances in the brain waves.

12. The sound generation method described in above 8, wherein the condition-evaluating process evaluates the condition of the mind and body by the ratio of $\alpha$-wave appearances in the brain waves.

13. The sound generation method described in any of above 3 to 10, wherein at least one of pulse wave, electrocardiograph, brain wave, electromyogram and respiration is used as the physiological signal.

14. A computer-readable storage medium for storing a program to execute at least one of the sound generation methods described in above 1 to 12, on a computer.

15. A computer-readable storage medium for storing a program to execute the sound generation method described in above 13, on a computer.

16. A stand-alone sound generation and playback apparatus comprising: means for measuring a physiological signal; a computer, which executes at least one of the sound generation methods described in above 1 to 12; means for playing a sound generated by the sound generation method; and means for measuring the condition of an individual informant who provides the physiological signal and listens to the sound.

17. A stand-alone sound generation and playback apparatus comprising: means for measuring a physiological signal; a computer, which executes the sound generation method described in above 13; means for playing a sound generated by the sound generation method; and means for measuring the condition of an individual informant who provides the physiological signal and listens to the sound.

18. A network-communicative sound generation and playback system comprising: a server computer, which executes at least one of the sound generation methods described in above 1 to 12; and means to be executed by a remote computer, which is connected to the server computer through a computer network, the means comprising means for measuring a physiological signal, which is necessary for the sound generation method, and means for playing a sound.

19. A network-communicative sound generation and playback system comprising: a server computer, which executes the sound generation method described in above 13; and means to be executed by a remote computer, which is connected to the server computer through a computer network, the means comprising means for measuring a physiological signal, which is necessary for the sound generation method, and means for playing a sound.

EFFECTS OF THE INVENTION

According to the sound generation method of the present invention, a sound is generated on the basis of basic information having chaos and/or fractal by converting the basic information into data that are numerically operable, by calculating a chaos and/or fractal characteristic of the basic information, and by applying a generation rule to the chaos and/or fractal data, which has been gained by the calculation.

According to the sound generation method of the present invention, a sound is generated to induce tranquility more effectively to the informant by changing the plane that cuts through the chaos attractor and by modifying the scaling width for extracting a fractal feature while the pulse wave and brain wave of the informant is being measured.

If the sound generation rule is modified interactively to the informant, then the sound generated can provide more relaxing effect to the informant.

Furthermore, if physiological information is used as basic information for the generation of a sound file, then a sound adapted to the individual who is providing the physiological information can be generated. For example, physiological information is taken when the individual feels well and at ease, and a sound can be generated on the basis of this physiological information to effect an improvement in his or her health condition or mood especially when his or her health deteriorates or he or she is in a gloomy mood. Like that for the purpose of calming down a baby, the baby is let to hear the sound used to hear in mother's womb before the birth, an individual is healed or encouraged by listening to a sound that is specific to the individual. This method is essentially different in sound quality from just letting an individual hear a sound from nature, which is generally referred to as a healing sound.

The condition of the mind and body is evaluated by the data of physiological signals that are classified into frequency bands. By repeating the modification and evaluation of the section and the scaling width, respectively, resultant evaluation values are led to converge, so that a more preferable or optimal condition be achieved.

According to the computer-readable storage medium on which a software program executing the sound generation method is stored, the above-mentioned sound generation method can be executed to generate a sound.

According to the stand-alone sound generation and playback apparatus, the above-mentioned sound generation method can be embodied in a single unit to generate a sound.

According to the network-communicative sound generation and playback system, the above-mentioned sound generation method is executed on a server computer to generate a sound on the basis of a physiological signal that is measured by a remote computer.

BRIEF DESCRIPTION OF THE DRAWINGS

FIG. 1 is an illustration describing the whole composition of a sound generation method, which generates a sound from basic information having a chaotic character, according to the present invention.

FIG. 2 is an illustration describing the whole composition of a sound generation method, which generates a sound from basic information having a fractal character, according to the present invention.

FIG. 3 shows the construction of a stand-alone sound generation and playback apparatus.

FIG. 4 is the construction of a network-communicative sound generation and playback system.

FIG. 5 is a graph of measured pulse waves.

FIG. 6 is another graph of measured pulse waves.

FIG. 7 is a three-dimensional graph of a calculated chaos attractor.

FIG. 8 is another three-dimensional graph of a calculated chaos attractor.

FIG. 9 is a schematic diagram showing plots on a sectional surface.

FIG. 10 is a graph describing a scaling conversion in a process of fractal feature extraction, for example, executed on pulse-wave data.

FIG. 11 is another graph describing a scaling conversion in a process of fractal feature extraction, for example, executed on pulse-wave data.

FIG. 12 is yet another graph describing a scaling conversion in a process of fractal feature extraction, for example, executed on pulse-wave data.

FIGS. 13 are graphs describing scaling conversions in a process of fractal feature extraction, for example, executed on pulse-wave data.

FIG. 14(a) is another graph describing scaling conversions in a process of fractal feature extraction, for example, executed on pulse-wave data, and FIG. 14(b) is an enlargement of part in FIG. 14(a).

FIG. 15(a) is an enlargement of part of the graph in FIG. 14(b), which describes a scaling conversion in a process of fractal feature extraction executed on pulse-wave data, and FIG. 15(b) is a further enlargement of part in FIG. 15(a).

FIG. 16(a) is a graph of pulse waves, and FIG. 16(b) is a three-dimensional graph of a chaos attractor, which is calculated by the scaling conversion of the pulse-wave graph.

FIG. 17(a) is a graph of pulse waves, and FIG. 17(b) is a three-dimensional graph of a chaos attractor, which is calculated by the scaling conversion of the pulse-wave graph.

FIG. 18(a) is a graph of pulse waves, and FIG. 18(b) is a three-dimensional graph of a chaos attractor, which is calculated by the scaling conversion of the pulse-wave graph.

FIGS. 19(a) to (c) are three-dimensional graphs of chaos attractors, each of which is calculated from respective pulse-wave data converted in scale, these graphs being representations of FIG. 16(b), FIG. 17(b) and FIG. 18(b).

FIGS. 20 are graphs of fractal features extracted directly from basic information, for example, pulse-wave data. FIG. 20(a) is a graph of chronological pulse-wave data, and FIG. 20(b) is a graph of data in the case where $|t_i - t_{i\pm k}| = 0.5$ second.

FIGS. 21 are graphs of fractal features extracted directly from basic information, for example, pulse-wave data. FIG. 21(a) is a graph of data in the case where $|t_i - t_{i\pm k}| = 0.05$ second, and

FIG. 21(b) is a graph of data in the case where $|t_i - t_{i\pm k}| = 0.005$ second.

FIG. 22 is a schematic diagram describing how to measure the feature extraction in the process of extracting a fractal feature directly from basic information, for example, pulse-wave data.

FIG. 23 is a schematic diagram describing the whole composition of a section-learning process according to the present invention.

FIG. 24 is a flow chart describing the section-learning process according to the present invention.

FIG. 25 is a diagram describing the whole composition of a scaling width learning process according to the present invention according to the present invention.

FIG. 26 is a flow chart describing the scaling width learning process.

FIG. 27 is a schematic diagram describing an environment where electroencephalography is performed.

FIG. 28 is a schematic diagram showing the positions of electrodes seen from above, which positions are in compliance with the international 10/20 standard for EEG placement.

FIG. 29 is a schematic diagram showing the same electrodes, which are positioned in compliance with the international 10/20 standard for EEG placement but are seen from the front.

FIG. 30 is a diagram describing the whole composition of a generation-rule learning process according to the present invention.

FIG. 31 is a flow chart describing a sound generation rule learning process for generating a sound from basic information that has a chaotic characteristic, according to the present invention.

FIG. 32 is a flow chart describing a sound generation rule learning process for generating a sound from basic information that has a fractal characteristic, according to the present invention.

FIG. 33(a) is a graph showing a fluctuation in the heartbeat, which is measured from a person who is listening to a sound, and FIG. 33(b) is a graph showing the result of an analysis of a neurological characteristic extracted from a physiological signal, for example, a fluctuation in the heartbeat shown in FIG. 33(a).

BEST-MODE EMBODIMENT OF THE INVENTION

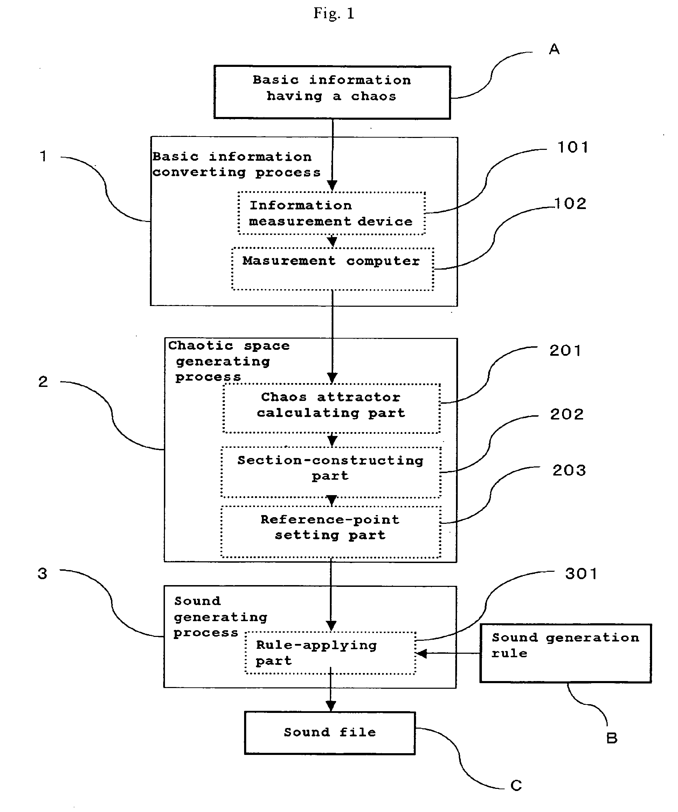

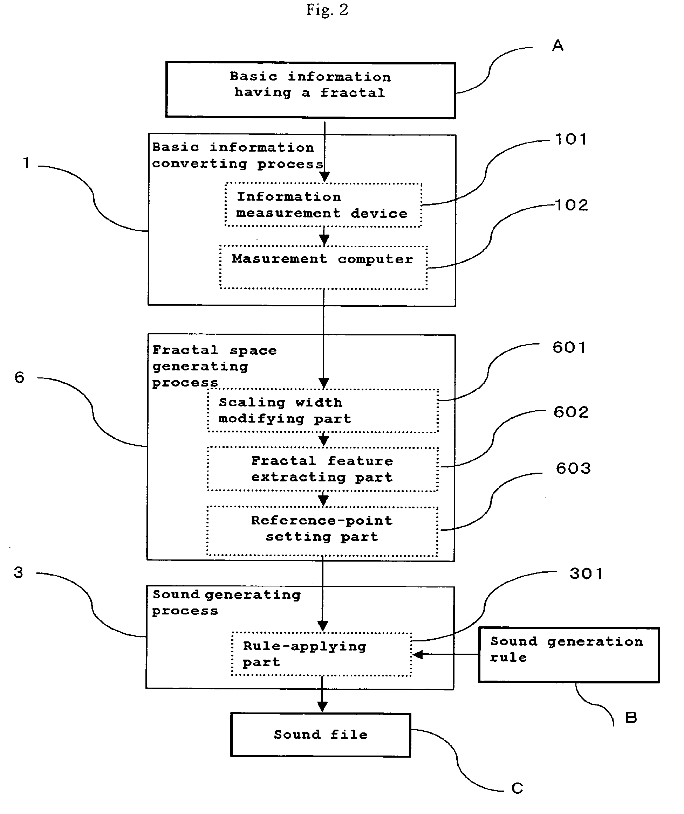

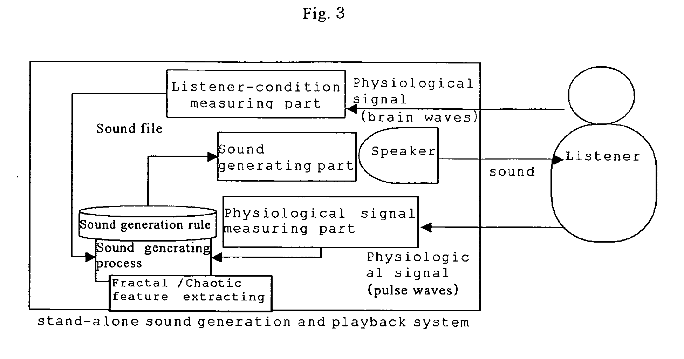

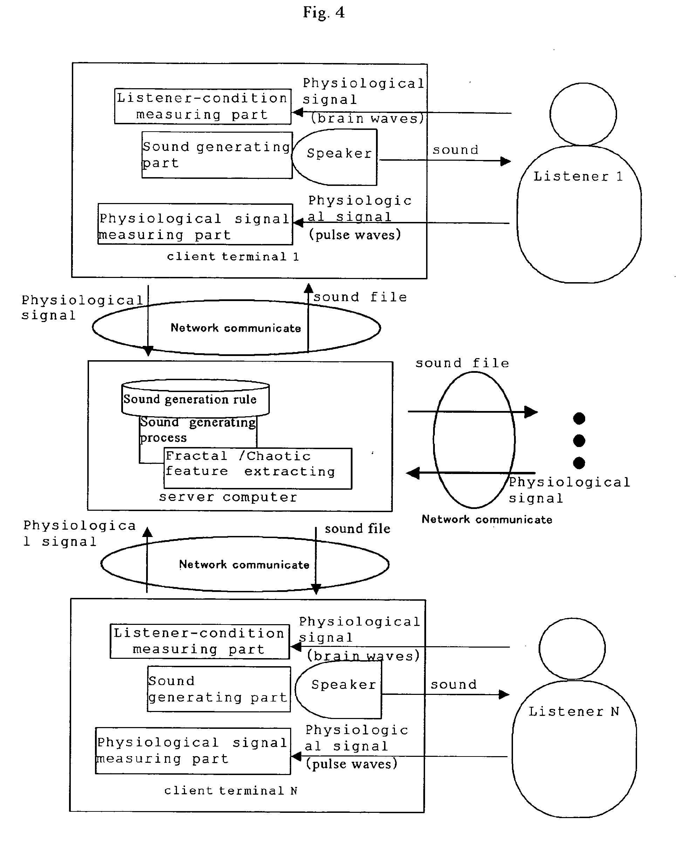

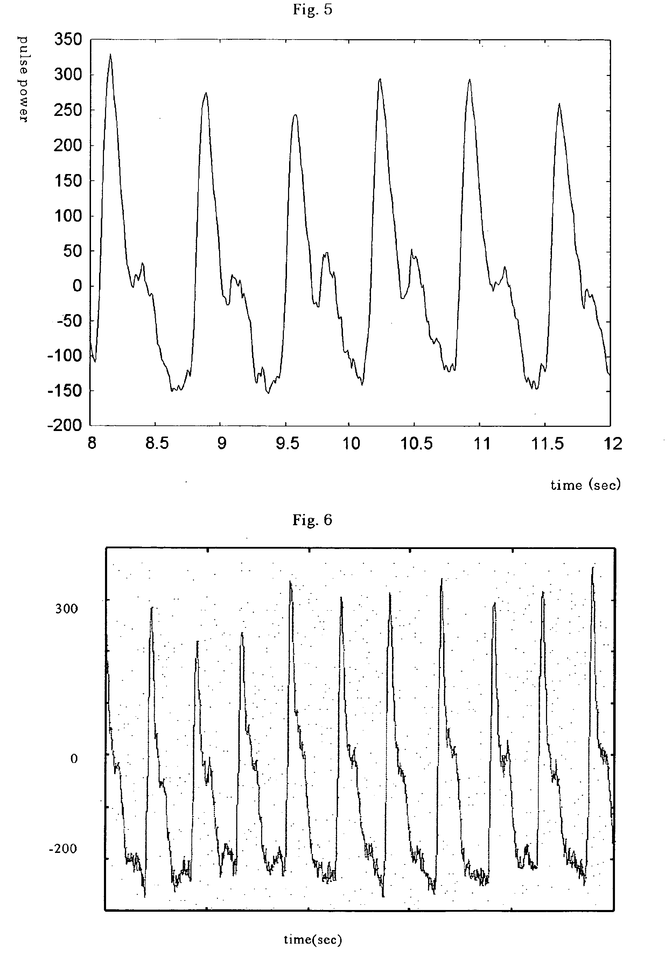

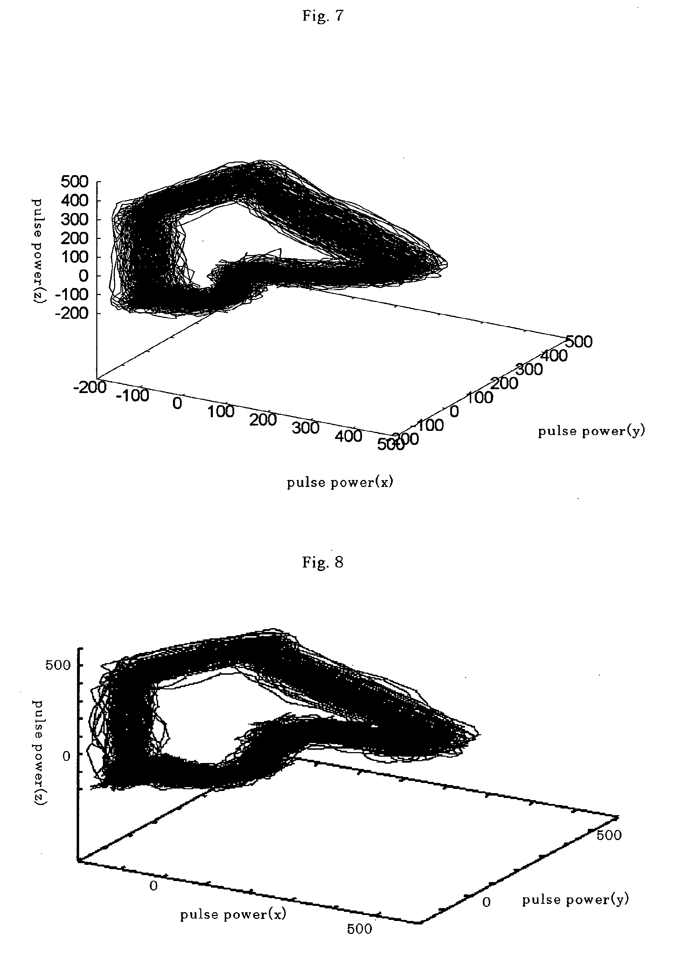

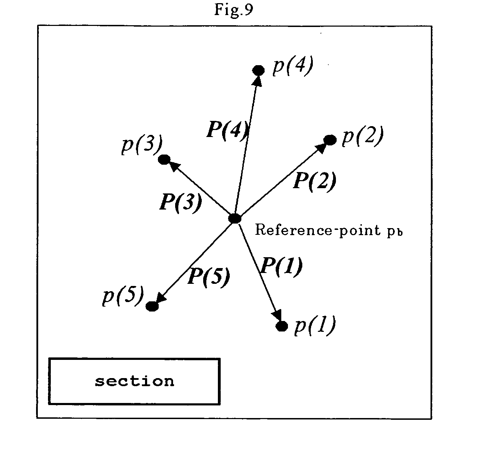

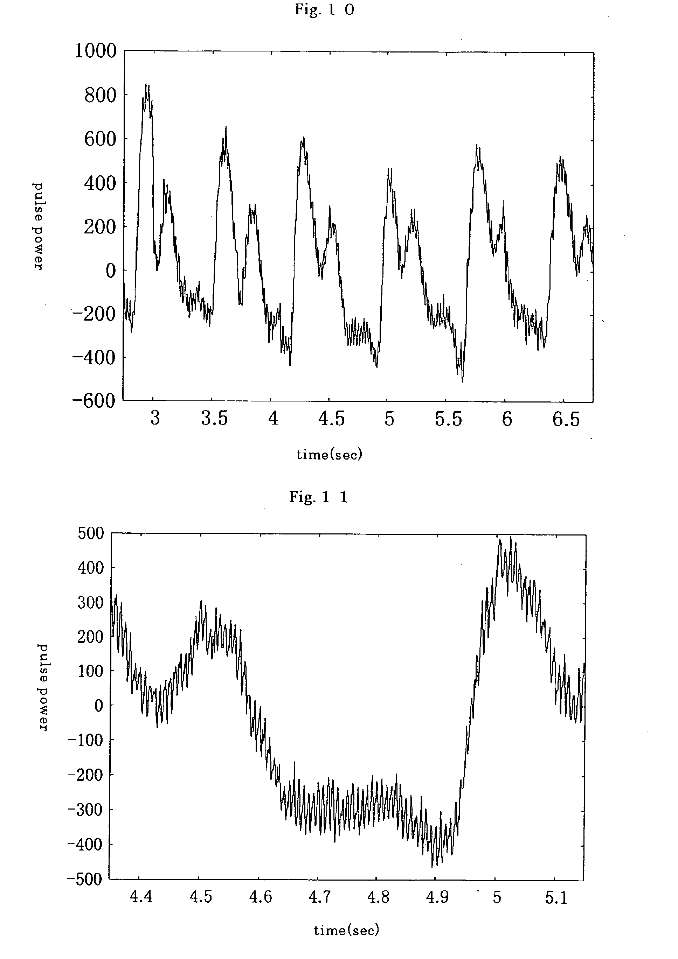

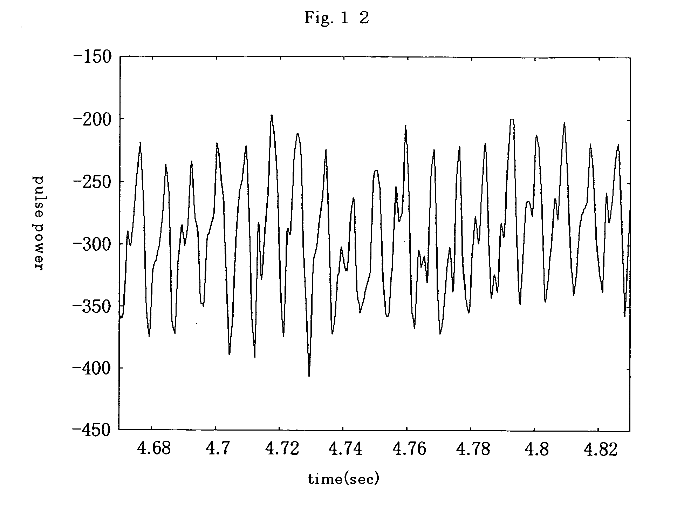

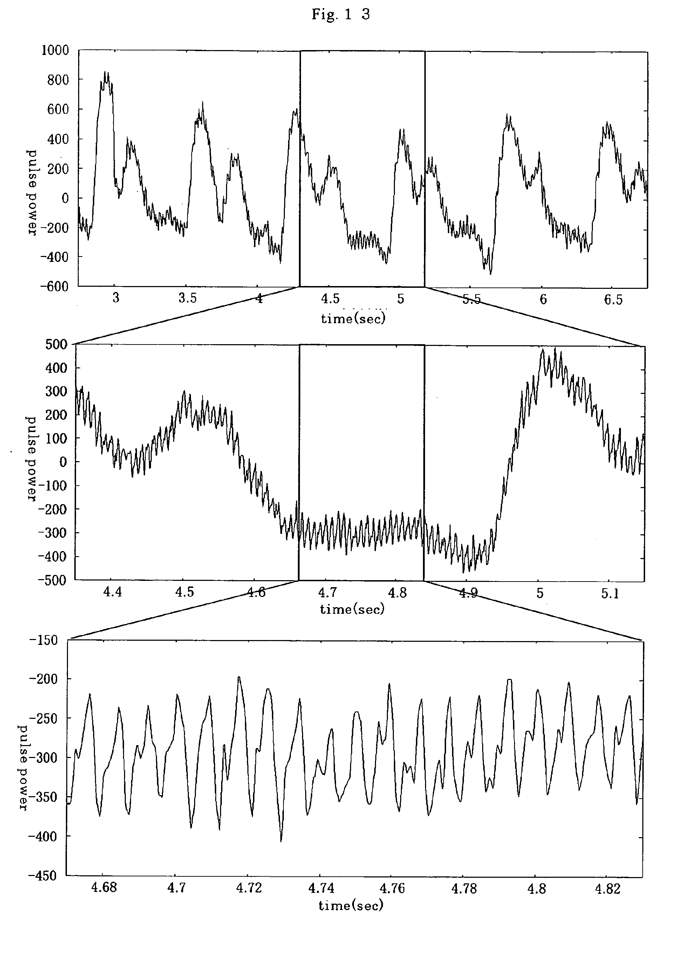

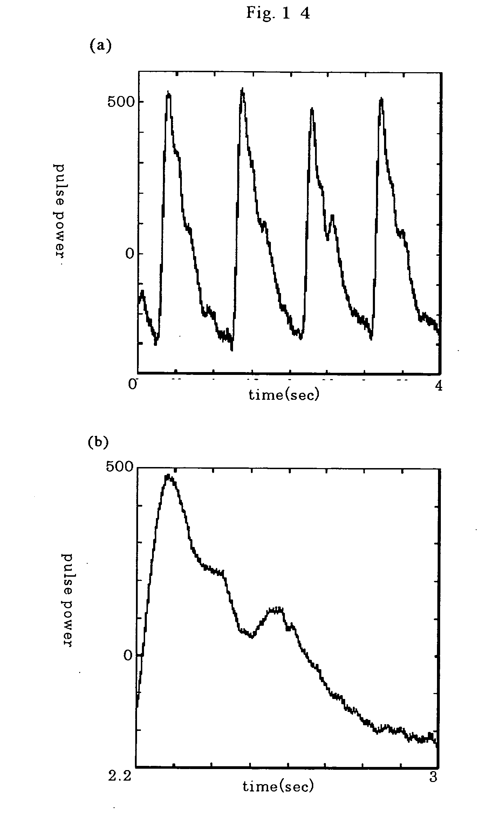

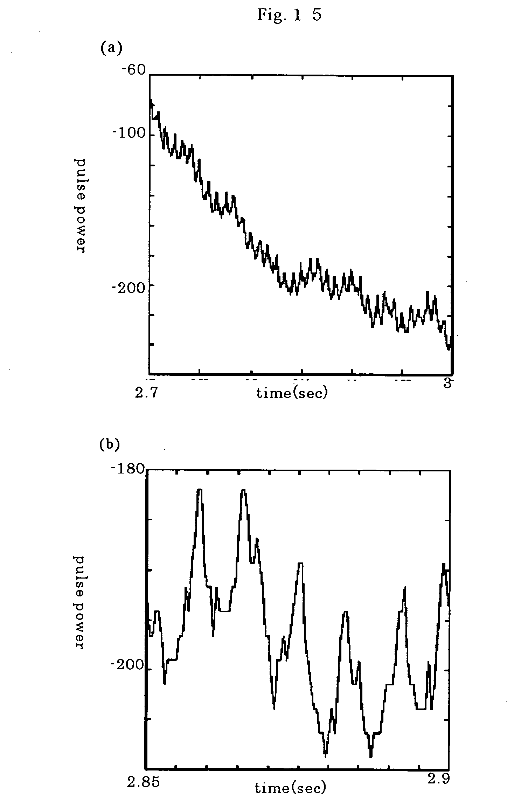

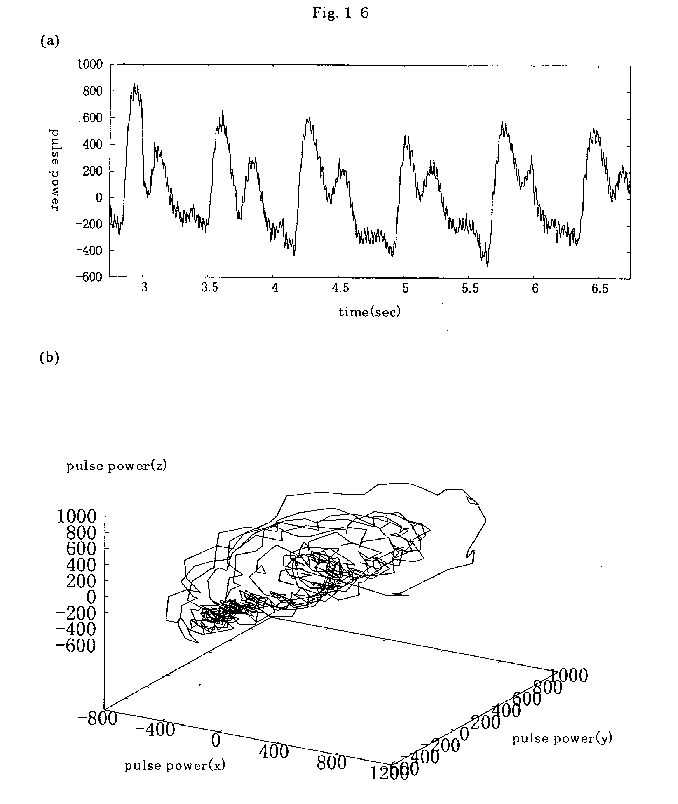

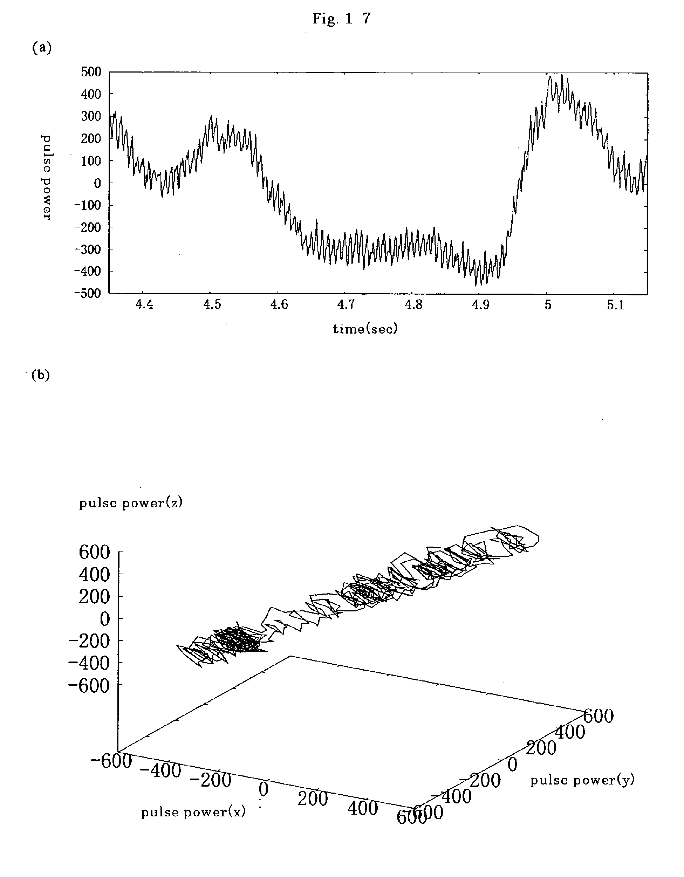

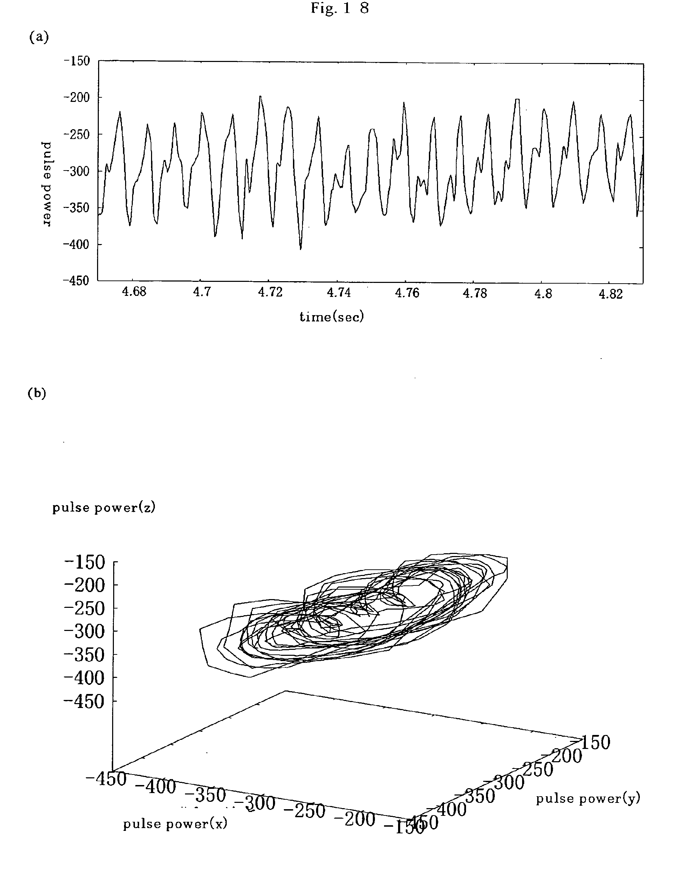

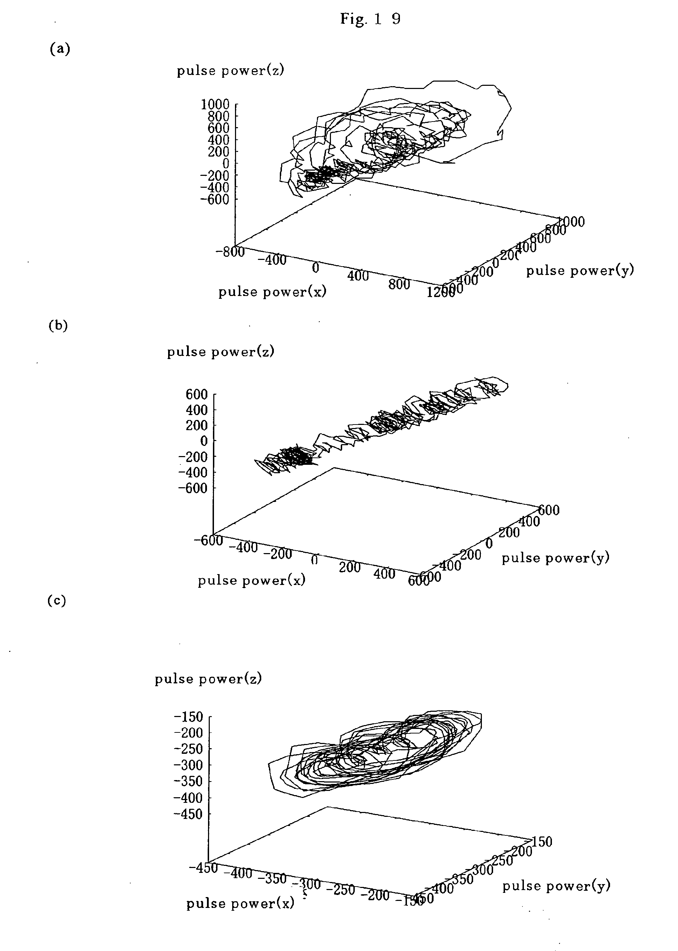

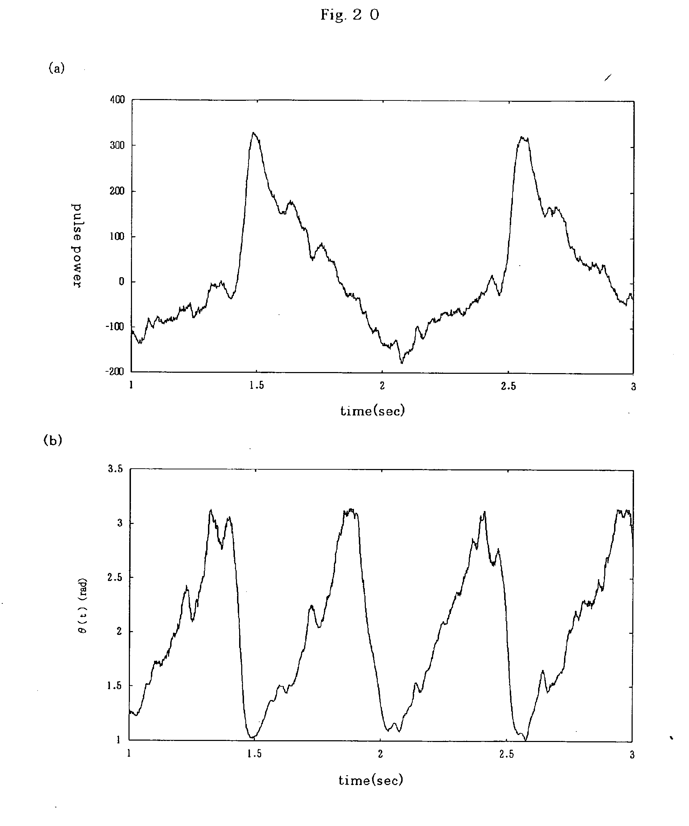

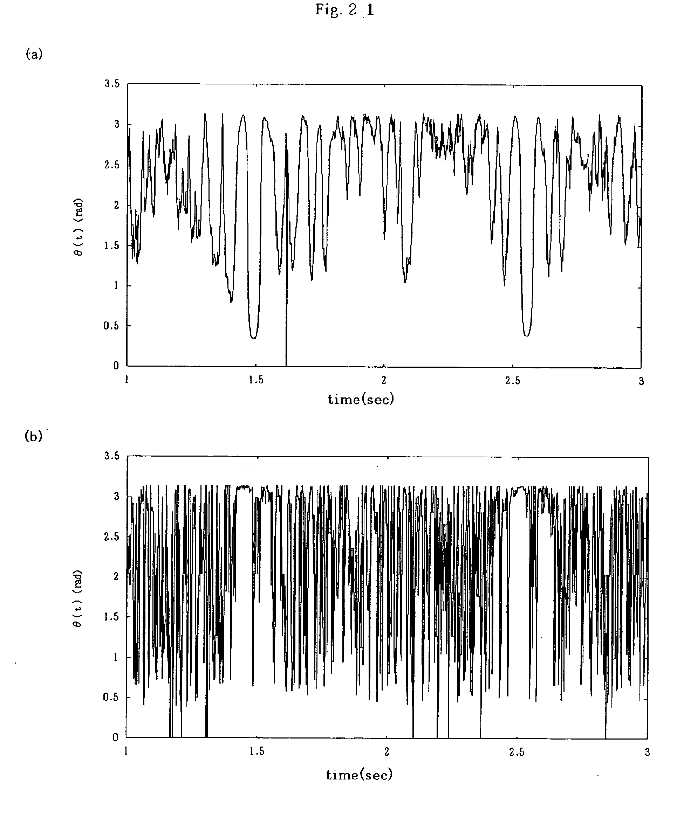

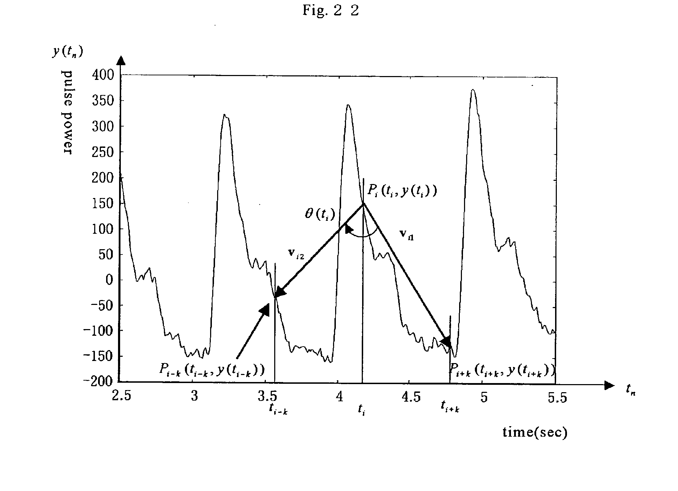

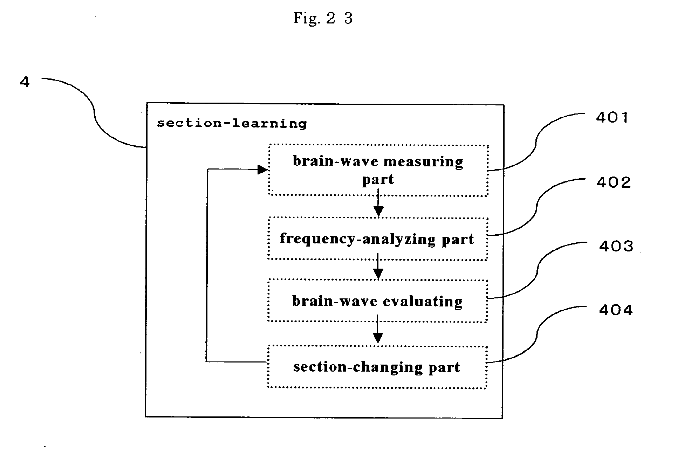

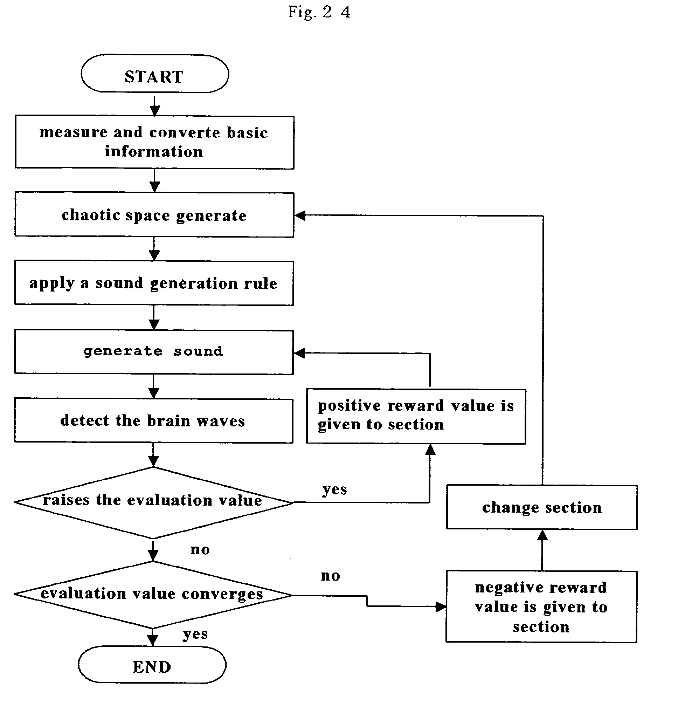

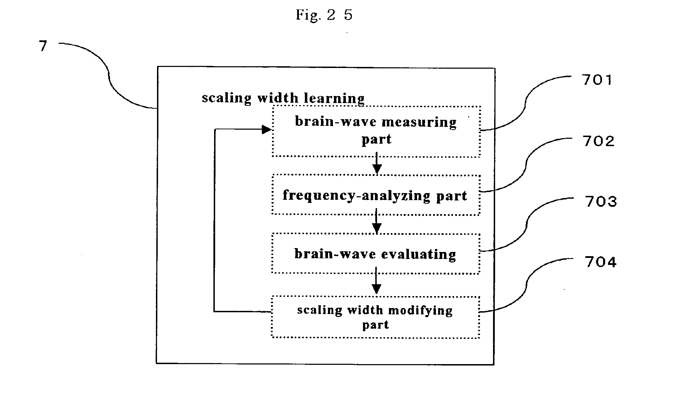

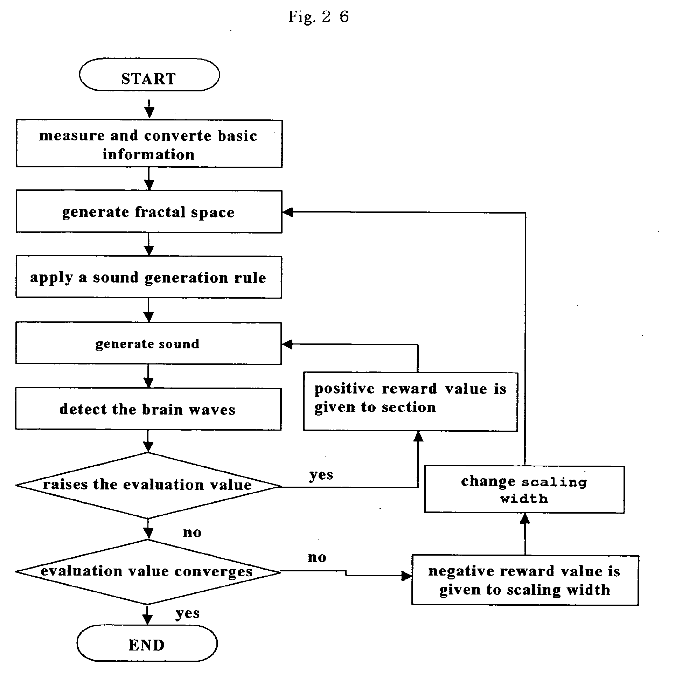

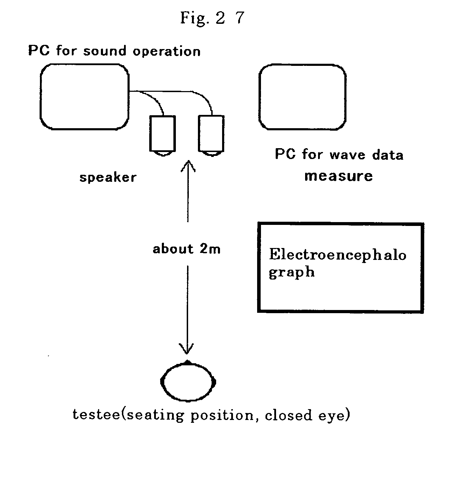

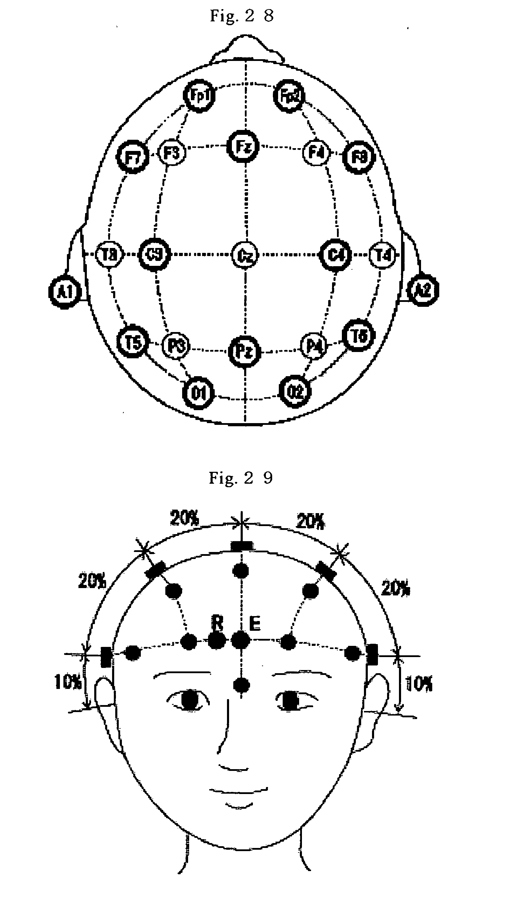

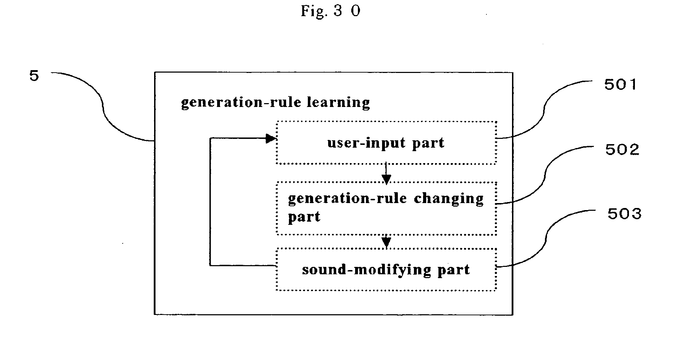

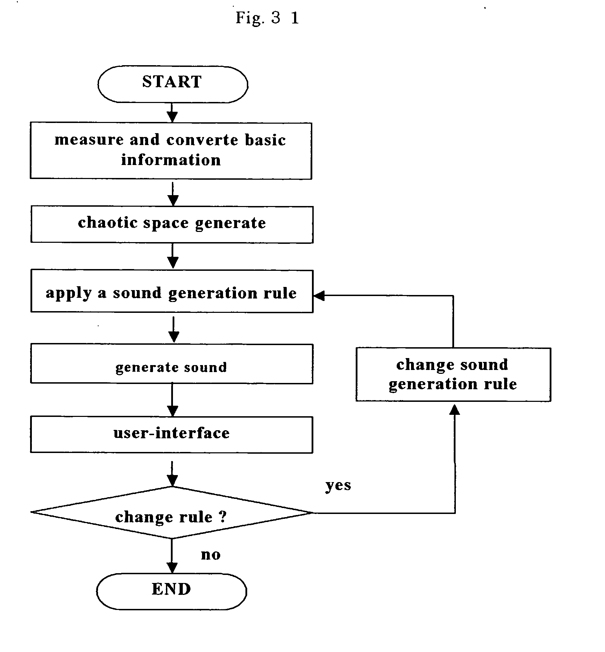

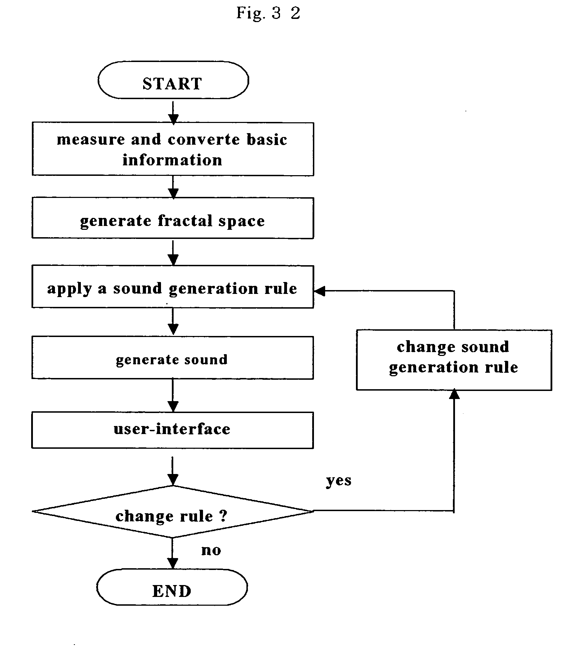

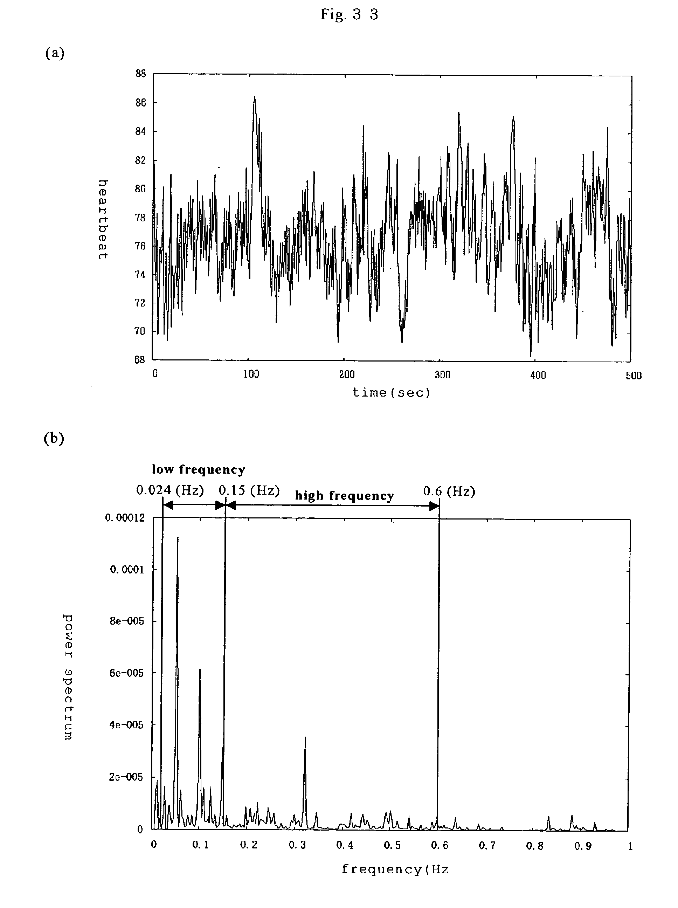

Now, preferred embodiments according to the present invention are described in reference to the drawings.
The above-mentioned "chaos" is a characteristic that seemingly shows irregular actions without periodicity or approximate periodicity even if the concerned system follows a deterministic equation. Also, the above-mentioned "fractal" refers to incorporation of self-similarity.
The above-mentioned "basic information" having chaotic and fractal characteristics are signals that originate, for example, from organisms, weather condition, and so on. In addition to physiological signals, examples are information that arises in the nature such as sound data like the murmur of a river, measurement data of the strength and/ or direction of winds, and vibration data measured with a seismograph.
The above-mentioned "physiological signal" can be any signal that is measured from a living body and can be chosen freely. Examples are brain waves, pulse waves measured from the pulsation, electrocardiogram, which shows chronological changes in electrical potential caused by the heart beat, electromyogram, which shows chronological changes in electrical potential caused by contractions of muscles, and expiration, which causes changes in the pressure or flow rate of the breathing through the mouth.
The above-mentioned "sound file" can be any data reproducible as a sound and can be in any type of file format. It can be, for example, in a PCM (Pulse Code Modulation) format that is used for sampling audio signals, or in a format that lists the frequencies and time intervals of a sound in a predetermined form. Furthermore, for example, a sound sampled specifically in standard MIDI format file (such a sound file is also referred to as a MIDI file or a SMF file) and PCM format can be compiled as a file (WAVE file).
As shown in FIG. 1, a generation method according to the present invention comprises a basic information converting part 1, where basic information A having a chaos and fractal is measured and converted into numerical data for computation, a chaotic space generating process 2, where a chaotic space is generated by calculating a chaos attractor, and a sound generating process 3, where a sound file C having a chaotic behavior is generated by applying a sound generation rule B.
The basic information converting part 1 includes a basic information conversion process that converts basic information A having a chaos and/or fractal into numerical data by an information measurement device 101 and then measures the basic information A, which has a chaos and fractal in time series, by a measurement computer 102.
As shown in FIG. 1 and FIG. 2, the sound generation method in this embodiment also includes a basic information converting part 1, where basic information A having a chaos and fractal is measured and converted into numerical data for computation, a fractal space generating process 6 which is generated by extracting a fractal feature, and a sound generating process 3, where a sound file C having a fractal behavior is generated by applying a sound generation rule B.
A computer-readable storage medium on which a program of sound generation method using a physiological signal according to the present invention is stored includes a program that makes a computer generate a sound by using a physiological signal, a physiological signal used in the sound generation, and a sound file that is produced by the sound generation.
Also, a sound generation and playback apparatus according to the present invention comprises a measurement of a physiological signal and a playback of a sound file in a unified model or in a network-communicative model.
As shown in FIG. 3, the unified model of sound generation and playback apparatus comprises a listener-condition measuring part, a physiological signal measuring part, a sound generating part and a sound playback part.
As shown in FIG. 4, the network model of sound generation and playback apparatus comprises a server computer that includes a sound generating part, and a client terminal, which is a computer located remotely and connected to the server computer through a network. The client terminal comprises a listener-condition measuring part, a physiological signal measuring part and a sound playback part. In this case, the network is used for the transmission of the listener-condition and the physiological signal, which are measured by the listener-condition measuring part and the physiological signal measuring part, to the sound generating part of the server computer or for the transmission of a sound file from the sound generating part of the server computer to the sound playback part of the client terminal.
The above-mentioned "listener-condition measuring part" measures the condition of the listener, which is used in section learning. This listener condition can be detected by any measuring method, for example, by taking electroencephalogram. Therefore, the listener-condition measuring part of this embodiment measures brain waves to detect the listener condition.
The above-mentioned "physiological signal measuring part" measures a physiological signal. Furthermore, it uses pulse waves as a physiological signal.
The above-mentioned "sound generating part" comprises a computer that can execute the sound generation method so that a sound is generated on the basis of the listener condition, which is detected by the listener-condition measuring part, and the physiological signal, which is measured by the physiological signal measuring part.

The above-mentioned "sound playback part" comprises a speaker and an amplifier, which plays the sound generated by the sound generating part.

Furthermore, the above-mentioned "network" can be of any kind and any form.

Moreover, the computer-readable storage medium, which stores the sound generation method program, and the sound generation and playback apparatus may use either the sound generation method that uses chaos or the method that uses fractal. Also, they may comprise both the sound generation methods, and either method may be selected for use as desired. Furthermore, sound files generated by these sound generation methods can be mixed into one file.

1. Sound generation from chaotic basic information

The above-mentioned chaotic space generating process 2 considers the measured basic information A having a chaos in time series as one-dimensional time series data and defines it as x(t). Here, t represents elapse of time. As examples of measured basic information A having a chaos in time series, FIG. 5 and FIG. 6 show graphs of pulse waves, respectively.

A chaos attractor calculating part 201 calculates a chaos attractor by using two given parameters of time delay $\tau$ (second) and embedded dimension m (dimension), so the chaos attractor becomes multivariate data of m dimension. The following equation 1 defines chaos attractors. For the measured pulse waves, a time delay of 0.1 second and an embedded dimension of 3 are applied. FIG. 7 and FIG. 8 show two examples of three-dimensional graph of chaos attractors calculated by the equation 1.

Equation 1

$$v_t = \left\{ x\left(t\right), x\left(t + \tau\right), \cdots, x\left(t + \left(m - 1\right)\tau\right)\right\}$$

By using Poincare mapping against the calculated chaos attractor, a section-constructing part 202 calculates minute deviations in the period of the chaos. This calculation cuts the orbit of solutions of the chaos attractor along a predetermined plane and finds point sequences p(i) in (m - 1) dimensions which are plotted in the section (Poincare section). Here, i is the chronological number that is assigned to each of the set of points of intersection.

A reference-point setting part 203 sets up a reference point $P_b$ at a predetermined position in the section. This reference point $P_b$ can be set at any given position and is used to determine a vector to a point where a chaos attractor intersects the section.

The rule-applying part 301 of the sound generating process 3 calculates the difference P(i) between the position vector $p_b$ of the reference point $p_b$ and the position vector p(i) of a point sequence p(i), which is a point of intersection of a chaos attractor in the section. The differences are calculated, for example as shown in FIG. 9, as vectors P(i) between the reference point $p_b$ and each of the points of intersection p(1) to p(5) in the section.

A sound is generated by combining the vectors P(i) to a sound generation rule B. In the sound generation, a minimum unit (phoneme) is determined with height n, length l and strength v, and the sound generated from the chaos attractors of m dimensions can be composed in a series of chords of at least m phonemes. The sound generation rule B is determined, for example, from the vector P(i) of the point sequence p(i) in the Poincare section by applying the following transformation equations 2 to 5.

Equation 2

$$S_k(i) = (n_k, l_k, v_k)$$

Equation 3

$$n_k = m(a,b), \quad a = \vec{P}_k(i)/r, \quad b = \vec{P}_k(i) \bmod r$$

Equation 4

$$l_k = \gamma\big(p(i-1)\big)$$

Equation 5

$$v_k = \left|\bar{P}(i)\right|$$

Here, the i in the $S_k(i)$ is the ordinal number of a phoneme in channel k, and the m (a, b) represents musical scale elements, the a being octave height, and the b being scale number. The r represents the number of phonemes in the chosen musical scale. Furthermore, the $P_k(i)$ represents the value of the element at number k in the vector P(i), and the mod represents the calculation of the remainder.

However, as a sound generation rule, rules other than the above-described one can be also applied. For example, the angle of deviation calculated from the two vectors P(i -1) and P(i) can be used as a sound generation rule, with the two vectors being determined from the points of intersection collected last time and those collected before the last. Any such element as length and angle that can be determined from the reference point and the points of intersection in the Poincare section can be used for this purpose. Moreover, by using these elements, effects of performance such as type of musical instrument (tone), code progression, vibrato, portamento and echo can be determined so that the generated sound will achieve a higher mental effect.

2. Sound generation from fractal basic information

As for calculating the above-mentioned fractal space generating process 6, there is a method for calculating an attractor every time when a scaling conversion is executed repeatedly on the measured basic information having a fractal in time series. Another method is that the fractal space is calculated directly from the measured basic information.

The fractal space generating process 6 considers the measured basic information A having a chronological fractal as a one-dimensional chronological data and defines it as x(t). Here, the t represents elapse of time. Two examples of the x(t) are shown in FIGS. 10 to 13 and FIG. 14(a), FIG. 14(b), FIG. 15(a) and FIG. 15(b). FIGS. 11 to 13 show part of the content in FIG. 10 in enlargement, respectively, and FIG. 14(b), FIG. 15(a) and FIG. 15(b) show part of the content in FIG. 14(a). These x(t) are determined by executing scaling conversions on the graphs of pulse waves, which are examples of measured basic information A having a fractal in time series, and by extracting a fractal feature, which shows self-similarity.

For example, FIGS. 13 show each of the results of 5-fold scale expansion operated twice in the chronological direction on one fifth (0.8 second) of the chronological data of the plethysmogram measured with a pulse-wave sensor for 4 seconds. FIGS. 16 to 19 show three-dimensional graphs of (b) attractors calculated by the equation 1 from pulse waves (a) in time series, respectively. The three attractor spaces, which have been achieved in this way, are then mixed with sound series on which the above-mentioned sound conversion rule has been applied to generate a sound that is based on the basic information having a fractal.

In addition, complex forms brought about by changes in the chronological data can be considered as changes in the angle of deviation, so that the changes in the angle of deviation can be observed at a plurality of appropriate scales to extract a fractal feature directly from the measured basic information.

For example, the change $\theta(t)$ of the angle of deviation is measured on the plethysmogram shown in FIG. 20 (a) by applying a deviation angle calculation method described in FIG. 22 while the range of $|t_i - t_{i\pm k}|$ is being changed. In this method, one point $P_i (t_i, y(t_i))$ in the basic information is determined as a reference for a certain period of time, and the angle $\theta(t_i)$ is defined by the two points that are determined at the previous period $P_{i-k} (t_{i-k}, y(t_{i-k}))$ and at the next period $P_{i+k} (t_{i+k}, y(t_{i+k}))$.

By making the range $|t_i - t_{i\pm k}|$ change from 0.5 to 0.005 (second), three types of changes $\theta(t)$ in the angle of deviation are obtained as shown in FIG. 20(a), FIG. 21(a) and FIG. 21(b). Each sequence $\theta(t)$ is replaced with the p (i) in the above-mentioned sound conversion rule to generate a sound series at the timing that is in harmony with the rhythm of the pulse. The three sound series generated in this way are then mixed to generate a sound, which is based on the fractal basic information.

3. Section learning

As shown in FIG. 23, the section learning 4 comprises a brain-wave measuring part 401, which measures the informant's brain waves, a frequency-analyzing part 402, which processes the brain waves into numerical data classified in frequency bands, a brain-wave evaluating part 403, which compares the numerical data of each frequency band with the others to evaluate the condition of the mind and body, and a section-changing part 404, which changes the plane that cuts through the chaos attractor in correspondence with the evaluation.

The section learning 4 plays the sound generated on the basis of basic information, for example, brain waves as described in the flow chart of FIG. 24, which details the section learning process. It detects the brain waves of the listener who is listening to the sound and evaluates his or her condition and then feeds this evaluation back, so that the section can be changed in correspondence with the evaluation. In this way, a more suitable sound can be generated.

Furthermore, as shown in FIG. 25, a scaling width learning 7 in this embodiment comprises a brain-wave measuring part 701, which measures the informant's brain waves, a frequency-analyzing part 702, which processes the brain waves into numerical data classified in frequency bands, a brain-wave evaluating part 703, which compares the numerical data of each frequency band with the others to evaluate the condition of the mind and body, and a scaling width modifying part 704, which modifies the scaling width used for extracting a fractal feature in correspondence with the evaluation.

4. Scaling width learning

The scaling width learning 7 plays the sound generated on the basis of basic information, for example, brain waves as described in the flow chart of FIG. 26, which details a scaling width learning process. Then, it detects the brain waves of the listener who is listening to the sound and evaluates his or her condition and feeds this evaluation back, so that the scaling width can be modified in correspondence with the evaluation. In this way, a more suitable sound can be generated.

The above-mentioned brain-wave measuring parts 401 and 701 measure brain waves with electrodes attached at a plurality of positions on the head of the informant and convert this information into data that are numerically operable.

The brain wave measurement method in this embodiment is in compliance with the international 10/20 standard for EEG placement, and twelve positions (Fp1, Fp2, F7, F8, Fz, C3, C4, Pz, T5, T6, O1 and O2) are adopted for the electrodes in the electroencephalography. In addition, plethysmogram, ophthalmogram and electrocardiogram are taken.

As shown in FIG. 27, which is a schematic diagram describing an environment where electroencephalography is performed, while brain waves are being measured, for example, a speaker set is installed for sound playback at a predetermined distance in front of the testee, who wears the electrodes used for brain wave measurement. In addition, a computer that executes the sound generation method is activated to generate a sound file and play it back through this speaker set. As shown in FIGS. 28 and 29, the electrodes are positioned in reference to the diagram of electrode placement of the international 10/20 standard for EEG placement.

The above-mentioned frequency-analyzing parts 402 and 702 classify the brain-wave data, which are measured, into frequency bands of $\delta$ waves (0.5 to 3 Hz), $\theta$ waves (3 to 7 Hz), $\alpha$-waves (7 to 13 Hz), $\beta$ waves (13 to 30 Hz) and $\gamma$ waves (30 to 40 Hz) and convert the information into numerical data by calculating a power spectrum for these waves. These numerical data are used in the analysis of the brain waves to understand the condition of the brain.

The above-mentioned brain-wave evaluating part 403 determines interrelations among the numerical data of the frequency bands, which have been classified by the above-mentioned analysis, for evaluating whether or not the informant feels at ease, and the results of the evaluation are accumulated as reward values. As an evaluation scale of tranquility, the ratio of the waves in each frequency band to the total waves and the spectral value of each frequency band, which indicates the strength of the waves in each frequency band, are used to understand general tendency for the evaluation of the condition of the brain. Then, a positive reward value is given to a section that raises the evaluation value while a negative reward value is given to a section that lowers the evaluation value.

Also, the above-mentioned brain-wave evaluating part 703 determines interrelations among the numerical data of the frequency bands, which have been classified by the above-mentioned analysis, for evaluating whether or not the informant feels at ease. As an evaluation scale of tranquility, the ratio of the waves in each frequency band to the total waves and the spectral value of each frequency band, which indicates the strength of the waves in each frequency band, are used to understand general tendency for the evaluation of the condition of the brain. Then, a positive reward value is given to a scaling width that raises the evaluation value while a negative reward value is given to a scaling width that lowers the evaluation value.

When the evaluation value decreases, the above-mentioned section-changing part 404 takes the reward values having accumulated until now into consideration for changing the section and continues the brain-wave measurement. If the evaluation value converges, then the learning is considered to have come to completion. Therefore, the section at present is set as an optimal section for the brain-wave measurement, and the brain-wave measurement is terminated.

Also, when the evaluation value decreases, the above-mentioned scaling width modifying part 704 takes the reward values having accumulated until now into consideration for changing the scaling width and continues the brain-wave measurement. If the evaluation value converges, then the learning is considered to have come to completion. Therefore, the scaling width at present is set as an optimal scaling width for the brain-wave measurement, and the brain-wave measurement is terminated.

As shown in FIG. 30, the generation-rule learning 5 in this embodiment comprises a user-input part 501, which has an interface that enables the system to talk with the informant, a generation-rule changing part 502, which changes the sound generation rule B in accordance with the input content, and a sound-modifying part 503, which generates a sound in correspondence with the change. FIG. 31 and FIG. 32 are flow charts, each describing a generation-rule learning process. As shown in FIGS. 31 and 32, by the generation-rule learning 5, the tone, volume and the like of a sound to be generated are modified.

As means for evaluating the condition of the mind and body of the listener, besides the brain waves, another method can also use measured data of physiological signals of the pulse and the heartbeat. In this case, the frequency spectrum is also observed for changes, to understand the activities of the autonomic nervous system. FIG. 33(a) is a graph showing fluctuations in the heartbeat, which are measured from a person who is listening to a sound. The frequency components of the fluctuations are separated into two groups of low frequency components (0.024 to 0.15 Hz) and high frequency components (0.15 to 0.6 Hz), and the ratio of the high frequency over low frequency contents is observed to measure the ratio of the activities of the parasympathetic nerve to those of the sympathetic nerve (this ratio is referred to as "nerve-descriptive characteristic") in the living body while he or she is listening to a sound attentively. FIG. 33(b) is a graph showing the result of an analysis of the nerve-descriptive characteristic, which is extracted from a physiological signal, in this case, fluctuations in the heartbeat shown in FIG. 33(a). When it is difficult to measure the brain waves of the listener, the value of the high frequency over low frequency is used to replace the evaluation value for the tranquility of the listener.

The above-mentioned user-input part 501 has an interface by which the informant listening to the sound can change the performing musical instrument and modify the ranges of the pitch, intensity and length of the sound, so that the informant's taste can be reflected.

The above-mentioned generation-rule changing part 502 applies to the generation rule the changes and modifications the informant has made by using the interface.

The above-mentioned section-changing part 503 generates a sound in compliance with the changed generation rule.

As shown in FIG. 1, a sound generation method using such a chaos attractor measures basic information A from an informant and converts it into data that are numerically operable, by a basic information converting part 1, and then, a chaos attractor is calculated from the data by a chaotic space generating process 2. Thereafter, the method hands the chaos attractor to a sound generating process 3, where a sound file C having a chaotic behavior is generated by applying a sound generation rule B.

As shown in FIG. 2, a sound generation method using a fractal feature measures basic information A from an informant and converts it into data that are numerically operable, by a basic information converting part 1, and then, a fractal feature is calculated from the data by a fractal space generating process 6. Thereafter, the method hands the fractal feature to a sound generating process 3, where a sound file C is generated by applying a sound generation rule B.

## Claims

1. A sound generation method comprising:

   a basic information converting process, where basic information having a chaos is converted to data that are numerically operable;
   a chaotic space generating process, where a chaotic space is generated by calculating a chaos attractor on the basis of the data, which have been converted by said basic information converting process; and
   a sound generating process, where a sound file is generated from the data in the chaotic space, which has been generated by said chaotic space generating process, in compliance with a predetermined sound generation rule.

2. A sound generation method comprising:

a basic information converting process, where basic information having a fractal is converted to data that are numerically operable;

a fractal space generating process, where a fractal space is generated by extracting a fractal feature on the basis of the data, which have been converted by said basic information converting process; and

a sound generating process, where a sound file is generated from the fractal space, which has been generated by said fractal space generating process, in compliance with a predetermined sound generation rule.

3. A sound generation method comprising:

a physiological signal converting process, where a signal chronologically generated from an individual informant is converted to data that are numerically operable;

a chaotic space generating process, where a chaotic space is generated by calculating a chaos attractor on the basis of the data, which have been converted by said physiological signal converting process; and

a sound generating process, where a sound file adapted for said informant is generated from the data in the chaotic space, which has been generated by said chaotic space generating process, in compliance with a predetermined sound generation rule.

4. A sound generation method comprising:

a physiological signal converting process, where a signal chronologically generated from an individual informant is converted to data that are numerically operable;

a fractal space generating process, where a fractal space is generated by extracting a feature of self-similarity on the basis of the data, which have been converted by said physiological signal converting process; and

a sound generating process, where a sound file adapted for said informant is generated from the fractal space, which has been generated by said fractal space generating process, in compliance with a predetermined sound generation rule.

5. The sound generation method as set forth in claim 3, wherein said physiological signal converting process comprises:

a physiological signal measuring process, which measures a physiological signal;

a frequency-analyzing process, which calculates the physiological signal data measured by said physiological signal measuring process as numerical data for a plurality of frequency bands; and

a sound generating process, which corresponds to a nerve-descriptive characteristic of the living body of said individual informant on a basis of said frequency-analyzing process.

6. The sound generation method as set forth in claim 4, wherein said physiological signal converting process comprises:

a physiological signal measuring process, which measures a physiological signal;

a frequency-analyzing process, which calculates the physiological signal data measured by said physiological signal measuring process as numerical data for a plurality of frequency bands; and

a sound generating process, which corresponds to a nerve-descriptive characteristic of the living body of said individual informant on a basis of said frequency-analyzing process.

7. The sound generation method as set forth in claim 3, wherein said chaotic space generating process comprises:

a condition-evaluating process, which evaluates the condition of the mind and body of said informant by comparing the numerical data, which have been calculated by said frequency-analyzing process from the nerve-descriptive characteristic of the living body of said individual informant; and

a section-changing process, which changes a plane that cuts through the chaos attractor, in correspondence with the evaluation by said condition-evaluating process.

8. The sound generation method as set forth in claim 4, wherein said fractal space generating process comprises:

a condition-evaluating process, which evaluates the condition of the mind and body of said informant by comparing the numerical data, which have been calculated by said frequency-analyzing process from the nerve-descriptive characteristic of the living body of said individual informant; and

a scaling width modifying process, which modifies the scaling width for extracting a fractal feature, in correspondence with the evaluation by said condition-evaluating process.

9. The sound generation method as set forth in claim 3, wherein said sound generating process comprises:

a condition-inputting process, which has an interface to enable communication with the informant providing said physiological signal so that conditions for the sound generation can be input; and
a generation-rule setting process, which sets said sound generation rule in compliance with the conditions input by said condition-inputting process, so that said sound generating process generates said sound file in compliance with the generation rule, which has been set by said generation-rule setting process.

10. The sound generation method as set forth in claim 4, wherein said sound generating process comprises:

a condition-inputting process, which has an interface to enable communication with the informant providing said physiological signal so that conditions for the sound generation can be input; and
a generation-rule setting process, which sets said sound generation rule in compliance with the conditions input by said condition-inputting process, so that said sound generating process generates said sound file in compliance with the generation rule, which has been set by said generation-rule setting process.

11. The sound generation method as set forth in claim 7, wherein said condition-evaluating process evaluates the condition of the mind and body by the ratio of $\alpha$-wave appearances in the brain waves.

12. The sound generation method as set forth in claim 8, wherein said condition-evaluating process evaluates the condition of the mind and body by the ratio of $\alpha$-wave appearances in the brain waves.

13. The sound generation method as set forth in any one of claims 3 ~ 10, wherein at least one of pulse wave, electrocardiograph, brain wave, electromyogram and respiration is used as said physiological signal.

14. A computer-readable storage medium for storing a program to execute at least one of the sound generation methods set forth in claims 1 to 12, on a computer.

15. A computer-readable storage medium for storing a program to execute the sound generation method set forth in claim 13, on a computer.

16. A stand-alone sound generation and playback apparatus comprising:

means for measuring a physiological signal;
a computer, which executes at least one of the sound generation methods set forth in claims 1 to 12;
means for playing a sound generated by said sound generation method; and
means for measuring the condition of an individual informant who provides said physiological signal and listens to said sound.

17. A stand-alone sound generation and playback apparatus comprising:

means for measuring a physiological signal;
a computer, which executes the sound generation method set forth in claim 13;
means for playing a sound generated by said sound generation method; and
means for measuring the condition of an individual informant who provides said physiological signal and listens to said sound.

18. A network-communicative sound generation and playback system comprising:

a server computer, which executes at least one of the sound generation methods set forth in claims 1 to 12; and
means to be executed by a remote computer, which is connected to said server computer through a computer network, said means comprising means for measuring a physiological signal, which is necessary for said sound generation method, and means for playing a sound.

19. A network-communicative sound generation and playback system comprising:

a server computer, which executes the sound generation method set forth in claim 13; and
means to be executed by a remote computer, which is connected to said server computer through a computer network, said means comprising means for measuring a physiological signal, which is necessary for said sound generation method, and means for playing a sound.

Fig. 1

A

Basic information
having a chaos

1

Basic information
converting process

101
Information
measurement device

102
Masurement computer

2

Chaotic space
generating
process

201
Chaos attractor
calculating part

202
Section-constructing
part

203
Reference-point
setting part

3

Sound
generating
process

301
Rule-applying
part

Sound generation
rule

B

Sound file

C

Fig. 2

A — Basic information having a fractal

1 — Basic information converting process
- 101 — Information measurement device
- 102 — Masurement computer

6 — Fractal space generating process
- 601 — Scaling width modifying part
- 602 — Fractal feature extracting part
- 603 — Reference-point setting part

3 — Sound generating process
- 301 — Rule-applying part

B — Sound generation rule

C — Sound file

Fig. 3

stand-alone sound generation and playback system

Fig. 4

Fig. 5

Fig. 6

Fig. 7

pulse power(z)

pulse power(y)

pulse power(x)

Fig. 8

pulse power(z)

pulse power(y)

pulse power(x)

Fig.9

Fig. 1 0

Fig. 1 1

Fig. 1 2

Fig. 1 3

Fig. 1 4

(a)

(b)

Fig. 1 5

(a)

(b)

Fig. 1 6

(a)

(b)

Fig. 1 7

(a)

(b)

Fig. 1 8

(a)

(b)

pulse power(z)

Fig. 1 9

(a)

pulse power(z)

(b)

pulse power(z)

(c)

pulse power(z)

Fig. 2 0

(a)

(b)

Fig. 2 1

(a)

(b)

Fig. 2 2

Fig. 2 3

4

section-learning — 401

brain-wave measuring part — 401

frequency-analyzing part — 402

brain-wave evaluating — 403

section-changing part — 404

Fig. 2 4

```
                    ┌──────────────┐
                    │    START     │
                    └──────┬───────┘
                           ↓
            ┌──────────────────────────────┐
            │  measure and converte basic   │
            │          information          │
            └──────────────┬───────────────┘
                           ↓
            ┌──────────────────────────────┐
            │    chaotic space generate     │←──────────────────┐
            └──────────────┬───────────────┘                    │
                           ↓                                     │
            ┌──────────────────────────────┐                    │
            │   apply a sound generation rule│                   │
            └──────────────┬───────────────┘                    │
                           ↓                                     │
            ┌──────────────────────────────┐    ┌────────────────────────────┐
            │        generate sound         │←───│                            │
            └──────────────┬───────────────┘    │                            │
                           ↓            ┌──────────────────────────────┐      │
            ┌──────────────────────────────┐    │  positive reward value is   │
            │      detect the brain waves   │    │     given to section        │
            └──────────────┬───────────────┘    └────────────┬───────────────┘
                           ↓                                  ↑
                      ╱─────────╲           yes               │
                  ╱─────────────────╲ ─────────────────────────
                  ╲  raises the      ╱                        ┌──────────────────────┐
                  ╲ evaluation value╱                         │    change section    │
                      ╲───────────╱                           └───────────┬──────────┘
                           ↓  no                                          ↑
                      ╱─────────╲              no             ┌──────────────────────────┐
                  ╱─────────────────╲ ─────────────────────── │   negative reward        │
                  ╲ evaluation value ╱                        │  value is given to        │
                  ╲    converges    ╱                         │      section              │
                      ╲───────────╱                           └──────────────────────────┘
                           ↓  yes
                    ┌──────────────┐
                    │     END      │
                    └──────────────┘
```

Fig. 2 5

7

701

scaling width learning

brain-wave measuring part

702

frequency-analyzing part

703

brain-wave evaluating

704

scaling width modifying part

Fig. 2 6

```
                    ┌─────────────┐
                    │    START    │
                    └──────┬──────┘
                           ↓
              ┌────────────────────────┐
              │ measure and converte   │
              │       basic            │
              │    information         │
              └───────────┬────────────┘
                          ↓
              ┌────────────────────────┐
              │  generate fractal space │←──────────────────────┐
              └───────────┬────────────┘                        │
                          ↓                                      │
              ┌────────────────────────┐                        │
              │ apply a sound           │                       │
              │  generation rule        │                       │
              └───────────┬────────────┘                        │
                          ↓                                      │
              ┌────────────────────────┐                        │
              │     generate sound      │←────────┐             │
              └───────────┬────────────┘          │             │
                          ↓                        │             │
              ┌────────────────────────┐   ┌──────────────────┐ │
              │  detect the brain waves │   │ positive reward  │ │
              └───────────┬────────────┘   │ value is given   │ │
                          ↓                │   to section     │ │
                      ◇◇◇◇◇◇◇◇◇◇           └─────────┬────────┘ │
              ◇ raises the evaluation value ◇─yes──────┘          │
                      ◇◇◇◇◇◇◇◇◇◇                       ┌─────────┴──────┐
                          ↓ no                          │ change scaling │
                      ◇◇◇◇◇◇◇◇◇◇                        │     width      │
              ◇ evaluation value converges ◇─no──┐      └────────┬───────┘
                      ◇◇◇◇◇◇◇◇◇◇                 ↓     ┌─────────┴──────┐
                          ↓ yes                        │ negative reward│
                    ┌─────────────┐                    │ value is given │
                    │     END     │                    │ to scaling width│
                    └─────────────┘                    └────────────────┘
```

36

Fig. 2 7

**PC for sound operation**

**speaker**

**PC for wave data measure**

**about 2m**

Electroencephalo graph

testee(seating position, closed eye)

Fig. 2 8

Fig. 2 9

Fig. 3 0

5

generation-rule learning

501

```
user-input part
        ↓
generation-rule changing
part
        ↓
sound-modifying part
```

502

503

Fig. 3 1

```
        ┌─────────────┐
        │    START    │
        └──────┬──────┘
               ▼
 ┌───────────────────────────┐
 │ measure and converte basic │
 │        information         │
 └─────────────┬─────────────┘
               ▼
 ┌───────────────────────────┐
 │   chaotic space generate   │
 └─────────────┬─────────────┘
               ▼
 ┌───────────────────────────┐        ┌──────────────────┐
 │ apply a sound generation   │◄───────│  change sound    │
 │          rule              │        │ generation rule  │
 └─────────────┬─────────────┘        └────────▲─────────┘
               ▼                                │
 ┌───────────────────────────┐                 │
 │       generate sound       │                 │
 └─────────────┬─────────────┘                 │
               ▼                                │
 ┌───────────────────────────┐                 │
 │       user-interface       │                 │
 └─────────────┬─────────────┘                 │
               ▼                                │
            ◇─────────◇           yes           │
          ◇ change rule ? ◇────────────────────┘
            ◇─────────◇
               │ no
               ▼
        ┌─────────────┐
        │     END     │
        └─────────────┘
```

Fig. 3 2

```
                        ┌──────────────┐
                        │    START     │
                        └──────┬───────┘
                               │
                               ▼
              ┌──────────────────────────────────┐
              │  measure and converte basic       │
              │           information             │
              └────────────────┬──────────────────┘
                               │
                               ▼
              ┌──────────────────────────────────┐
              │       generate fractal space      │
              └────────────────┬──────────────────┘
                               │
                               ▼
              ┌──────────────────────────────────┐          ┌─────────────────────┐
              │   apply a sound generation rule   │◄─────────│   change sound      │
              └────────────────┬──────────────────┘          │  generation rule    │
                               │                             └──────────▲──────────┘
                               ▼                                        │
              ┌──────────────────────────────────┐                     │
              │          generate sound           │                     │
              └────────────────┬──────────────────┘                     │
                               │                                        │
                               ▼                                        │
              ┌──────────────────────────────────┐                     │
              │          user-interface           │                     │
              └────────────────┬──────────────────┘                     │
                               │                              yes       │
                               ▼                                        │
                       ◇─────────────────◇─────────────────────────────┘
                       │   change rule ?  │
                       ◇─────────────────◇
                               │ no
                               ▼
                        ┌──────────────┐
                        │     END      │
                        └──────────────┘
```

Fig. 3 3

(a)

(b)

**EP 1 559 444 A1**

<table>
<tr><td colspan="2"><b>INTERNATIONAL SEARCH REPORT</b></td><td>International application No.<br><br>PCT/JP03/16001</td></tr>
</table>

A. CLASSIFICATION OF SUBJECT MATTER
Int.Cl⁷ A61M21/02

According to International Patent Classification (IPC) or to both national classification and IPC

B. FIELDS SEARCHED

Minimum documentation searched (classification system followed by classification symbols)
Int.Cl⁷ A61M21/00-21/02, A61B5/00

Documentation searched other than minimum documentation to the extent that such documents are included in the fields searched
Jitsuyo Shinan Koho      1922-1996   Jitsuyo Shinan Toroku Koho   1996-2004
Kokai Jitsuyo Shinan Koho  1971-2004   Toroku Jitsuyo Shinan Koho   1994-2004

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | JP 7-51374 A (Pioneer Electronic Corp.),<br>28 February, 1995 (28.02.95),<br>Full text; all drawings<br>& EP 638282 A1      & US 5613498 A | 1-19 |
| A | JP 9-322943 A (Matsushita Electric Industrial Co., Ltd.),<br>16 December, 1997 (16.12.97),<br>Full text; all drawings<br>(Family: none) | 1,3,5,7,9,<br>11,13-19 |
| A | JP 2001-283134 A (Sharp Corp.),<br>12 October, 2001 (12.10.01),<br>Full text; all drawings<br>(Family: none) | 1,3,5,7,9,<br>11,13-19 |

☒ Further documents are listed in the continuation of Box C.      ☐ See patent family annex.

| | |
|---|---|
| * Special categories of cited documents:<br>"A" document defining the general state of the art which is not considered to be of particular relevance<br>"E" earlier document but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search<br>16 April, 2004 (16.04.04) | Date of mailing of the international search report<br>11 May, 2004 (11.05.04) |
|---|---|
| Name and mailing address of the ISA/<br>Japanese Patent Office | Authorized officer |
| Facsimile No. | Telephone No. |

Form PCT/ISA/210 (second sheet) (July 1998)

43

**EP 1 559 444 A1**

## INTERNATIONAL SEARCH REPORT

| International application No. |
| --- |
| PCT/JP03/16001 |

C (Continuation). DOCUMENTS CONSIDERED TO BE RELEVANT

| Category* | Citation of document, with indication, where appropriate, of the relevant passages | Relevant to claim No. |
| --- | --- | --- |
| A | US 6461316 B1 (Richard H. Lee), 08 October, 2002 (08.10.02), Full text; all drawings & CN 1217943 A | 1,3,5,7,9, 11,13-19 |
| A | US 6135944 A (Zebedee Research, Inc.), 24 October, 2000 (24.10.00), Full text; all drawings (Family: none) | 2,4,6,8,10, 12-19 |

Form PCT/ISA/210 (continuation of second sheet) (July 1998)